# EUROPEAN PATENT SPECIFICATION

(11) **EP 2 596 797 B1**
(45) Date of publication and mention of the grant of the patent: **21.10.2015**
(21) Application number: 11190304.3
(22) Date of filing: 23.11.2011
(51) Int. Cl.: A61K 35/74, A61K 36/06, A61P 15/02, A61K 9/48

(54) **A vaginal capsule**
Vaginalkapsel
Capsule vaginale

(43) Date of publication of application: 29.05.2013
(73) Proprietor: Biolatte Oy, 20321 Turku (FI)
(72) Inventor: Delatte, Yves, 20460 Turku (FI)
(74) Representative: Turun Patenttitoimisto Oy

(56) References cited:
- WO-A1-2010/054439
- WO-A2-03/080813
- US-A1- 2004 071 679
- US-A1- 2010 151 026
- US-A1- 2011 189 132
- DATABASE WPI Week 201050 Thomson Scientific, London, GB; AN 2010-E56915 XP002674763, & CN 101 690 734 A (XIAN ZHENGHAO BIOLOGICAL PHARM CO LTD) 7 April 2010 (2010-04-07)
- R. HERBRECHT ET AL: "Saccharomyces cerevisiae Fungemia: An Adverse Effect of Saccharomyces boulardii Probiotic Administration", CLINICAL INFECTIOUS DISEASES, vol. 40, no. 11, 1 June 2005 (2005-06-01), pages 1635-1637, XP55024023, ISSN: 1058-4838, DOI: 10.1086/429926
- DATABASE WPI Week 201166 Thomson Scientific, London, GB; AN 2011-K08508 XP002674764, & CN 102 120 045 A (GROWING BIOLOGICAL ENG SHANGHAI CO LTD) 13 July 2011 (2011-07-13)
- LIBBY EDWARDS: "The diagnosis and treatment of infectious vaginitis", DERMATOLOGIC THERAPY, vol. 17, no. 1, 1 January 2004 (2004-01-01), pages 102-110, XP55024024, ISSN: 1396-0296, DOI: 10.1111/j.1396-0296.2004.04010.x

## Description

The present invention relates to a means of treatment of vaginitis. The invention also relates to a capsule comprising at least one type of yeast and one type of bacteria. The capsule is useful for treatment of vaginitis.

Vaginitis is a disease of the vagina. It is usually due to infection. The three main kinds of vaginitis are bacterial vaginosis, vaginal candidiasis, and trichomoniasis. It is known to treat vaginitis with antibiotics and/or probiotic products. Some probiotic products are probiotic food supplements containing starches. There are however some problems associated with these products, such as the antibiotics disturbing the vaginal flora and the probiotic may have components that could be allergenic to.

There is therefore a need to provide a treatment of vaginitis, that does not have these drawbacks. Such treatment preferably is non-allergenic and effective even with few applications.

The present invention relates to a capsule made of a polysaccharide metabolisable in human body temperature. The capsule contains Saccharomyces boulardii in an amount of 10E8 - 10E9 units/g, and at least one bacteria selected from the group consisting of Lactobacillus crispatus, Lactobacillus jensinii, Lactobacillus gasseri and Enterococcus faecium, in an amount of 10E8 - 10E9 cfu/g. cfu stands for colony-forming unit and is the recognised measure of viable bacterial numbers.

The bacteria and yeast used in the present invention are bacteria and yeast naturally present in the human organism, such as vaginal and/or colonic bacteria. Saccharomyces boulardii is present since untreated vaginal infections are often followed by mucosal infections which need to be prevented. The bacteria E.faecium, a lactobacillus bacteria selected from the group consisting of L.cryspatus, L.jensinii and L.gasseri, and Sc.boulardii have been found to have a synergestic effecf as they work symbiotically.

Therefore, according to a preferred embodiment of the invention, the capsule contains, in addition to Saccharomyces boulardii, at least one Lactobacillus bacteria selected from the group consisting of Lactobacillus crispatus, Lactobacillus jensinii and Lactobacillus gasseri, and Enterococcus faecium, each in an amount of 10E8 - 10E9 cfu/g. Preferred amounts of these bacteria are 10E9 - 9 x 10E9 cfu/g.

The amounts of each bacteria can be the same or different, or the amount of two bacteria can be identical but different from the amount of the third bacteria.

According to an embodiment of the invention, amount of each bacteria Lactobacillus crispatus, Lactobacillus jensinii, Lactobacillus gasseri and/or Enterococcus faecium is, independently from each other, 2 x 10E9 - 9 x 10E9 cfu/g. The amount of Saccharomyces boulardii can be 3 x 10E9 - 9 x 10E9 units/g.

At the moment of making the invention, it has been found that the most preferred amount of any of the bacteria used is 3 x 10E9 - 5 x 10E9 cfu/g.

The composition according to the present invention is in a capsule that itself becomes the nutrient for the bacteria. The capsule is made of a polysaccharide, that metabolises at body temperature (around 37 ºC). The metabolisation is typically essentially completed within 10 minutes of the application of the capsule. The material of the capsule naturally needs to be allergen-free for the user.

Polysaccharides are long carbohydrate molecules, of repeated monomer units joined together by glycosidic bonds. Some examples of polysaccharides are starch, glycogen, cellulose, chitin, dextran, pentosans, fructans and pullulans.

The preferred polysaccharide is pullulan or one of its derivatives. Also a pullulan-type polysaccharide may be used. Pullulan is a polysaccharide polymer consisting of maltotriose units, also known as α-1,4- ;α-1,6-glucan. Pullulan is produced from starch by the fungus *Aureobasidium pullulans.* Pullulans are non-toxic, hallal and kosher, edible, biodegradable and biocompatible. They have the general formula of (C₆H₁₀O₅)ₙ·H₂O, three glucose units in maltotriose being connected by an α-1,4 glycosidic bond, whereas consecutive maltotriose units are connected to each other by an α-1,6 glycosidic bond. The formula is thus as follows.

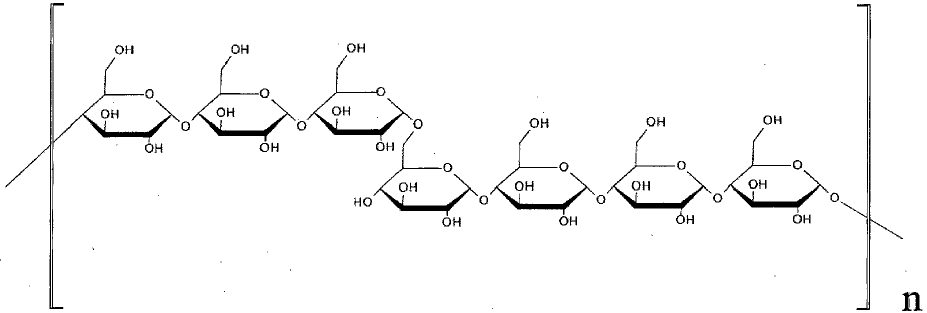

When the capsule material has metabolised, its proteins are used by the bacteria as nutrients.

The capsules are free of any known allergens and free of proteins derived from those allergens, thus making the product safe to a vast majority of people.

The size of the capsule is the commonly pharmaceutical standard size O or size OO. It can vary between 5-20 mm in lenght and 5-10 mm in diameter. The capsules preferably have either a spherical or longitudinal shape, and preferably without any sharp edges, in order to facilitate their administration.

The capsules can be administered in an amount of 1-2 capsules/day, but the importance is more in the duration and continuity of the treatment rather than the number of capsules taken at one time or per day. The preferred administration mode is local.

The invention further relates to a composition consisting of Saccharomyces boulardii in an amount of 10E8 - 10E9 units/g and at least one bacteria selected from the group consisting of Lactobacillus crispatus, Lactobacillus jensinii, Lactobacillus gasseri and Enterococcus faecium, in an amount of 10E8 - 10E9 cfu/g, for use as a means for treating vaginitis.

The invention yet further relates to a composition consisting of Saccharomyces boulardii in an amount of 10E8 - 10E9 units/g and at least one bacteria selected from the group consisting of Lactobacillus crispatus, Lactobacillus jensinii, Lactobacillus gasseri and Enterococcus faecium, in an amount of 10E8 - 10E9 cfu/g, for use as a medicine for treating vaginitis.

Experiments have been carried out and it has been found that in 80 % of the cases, a single or a few applications are sufficient to treat a common vaginitis.

## Claims

1. A capsule made of a polysaccharide metabolisable in human body temperature, useful for treatment of vaginitis, containing
- Saccharomyces boulardii in an amount of 10E8 - 10E10 units/g, and
- at least one bacteria selected from the group consisting of Lactobacillus crispatus, Lactobacillus jensinii, Lactobacillus gasseri and Enterococcus faecium in an amount of 10E8 - 10E9 cfu/g.

2. Capsule according to claim 1, **characterised in that** it contains
- at least one Lactobacillus bacteria selected from the group consisting of Lactobacillus crispatus, Lactobacillus jensinii and Lactobacillus gasseri, and
- Enterococcus faecium,
both in an amount of 10E8 - 10E9 cfu/g.

3. Capsule according to any one of the previous claims, **characterised in that** the amount of Saccharomyces boulardii is 2 x 10E9 - 9 x 10E9 units/g.

4. Capsule according to any one of the previous claims, **characterised in that** amount of Lactobacillus crispatus, Lactobacillus jensinii and/or Lactobacillus gasseri is 2 x 10E9 - 9 x 10E9 cfu/g.

5. Capsule according to any one of the previous claims, **characterised in that** amount of Enterococcus faecium is 2 x 10E9 - 9 x 10E9 cfu/g.

6. Capsule according to any one of the previous claims, **characterised in that** said polysaccharide is pullulan or a derivative of pullulan.

7. A composition consisting of Saccharomyces boulardii in an amount of 10E8 - 10E9 units/g and at least one bacteria selected from the group consisting of Lactobacillus crispatus, Lactobacillus jensinii, Lactobacillus gasseri and Enterococcus faecium, in an amount of 10E8 - 10E9 cfu/g, for use as a means for treating vaginitis.

## Patentansprüche

1. Kapsel, die aus einem Polysaccharid hergestellt ist, das bei menschlicher Körpertemperatur metabolisierbar ist, die zur Behandlung von Vaginitis verwendbar ist, enthaltend
- Saccharomyces boulardii in einer Menge von 10E8 - 10E10 Einheiten/g und
- wenigstens ein Bakterium, ausgewählt aus der Gruppe, bestehend aus Lactobacillus crispatus, Lactobacillus jensinii, Lactobacillus gasseri und Enterococcus faecium, in einer Menge von 10E8 - 10E9 kbE/g.

2. Kapsel gemäß Anspruch 1, **dadurch gekennzeichnet, dass** sie enthält
- wenigstens ein Lactobacillus -Bakterium ausgewählt aus der Gruppe, bestehend aus Lactobacillus crispatus, Lactobacillus jensinii und Lactobacillus gasseri, und
- Enterococcus faecium,
beide in einer Menge von 10E8 - 10E9 kbE/g.

3. Kapsel gemäß einem der vorangehenden Ansprüche, **dadurch gekennzeichnet, dass** die Menge an Saccharomyces boulardii 2 x 10E9 - 9 x 10E9 Einheiten/g ist.

4. Kapsel gemäß einem der vorangehenden Ansprüche, **dadurch gekennzeichnet, dass** die Menge an Lactobacillus crispatus, Lactobacillus jensinii und/oder Lactobacillus gasseri 2 x 10E9 - 9 x 10E9 kbE/g ist.

5. Kapsel gemäß einem der vorangehenden Ansprüche, **dadurch gekennzeichnet, dass** die Menge an Enterococcus faecium 2 x 10E9 - 9 x 10E9 kbE/g ist.

6. Kapsel gemäß einem der vorangehenden Ansprüche, **dadurch gekennzeichnet, dass** das Polysaccharid Pullulan oder ein Derivat von Pullulan ist.

7. Zusammensetzung, bestehend aus Saccharomyces boulardii in einer Menge von 10E8 - 10E9 Einheiten/g und wenigstens einem Bakterium, ausgewählt aus der Gruppe, bestehend aus Lactobacillus crispatus, Lactobacillus jensinii, Lactobacillus gasseri und Enterococcus faecium, in einer Menge von 10E8 - 10E9 kbE/g, zur Verwendung als Mittel zur Behandlung von Vaginitis.

## Revendications

1. Capsule faite d'un polysaccharide métabolisable à la température du corps humain, utile pour le traitement des vaginites, contenant :
- Saccharomyces boulardii en une quantité de 10E8-10E10 unités/g et
- au moins une bactérie choisie dans le groupe consistant en Lactobacillus crispatus, Lactobaccillus jensinii, Lactobaccillus gasseri et Enterococcus faecium dans une quantité de 10E8-10E9 ufc/g.

2. Capsule selon la revendication 1, **caractérisée en ce qu'**elle contient :
- au moins une bactérie choisie dans le groupe consistant en Lactobacillus crispatus, Lactobaccillus jensinii et Lactobaccillus gasseri et
- Enterococcus faecium
chacun dans une quantité de 10E8-10E9 ufc/g.

3. Capsule selon l'une quelconque des revendications précédentes, **caractérisée en ce que** la quantité de Saccharomyces boulardii est de 2x10E9 - 9x10E9 unités/g.

4. Capsule selon l'une quelconque des revendications précédentes **caractérisée en ce que** la quantité de Lactobacillus crispatus, Lactobaccillus jensinii et/ou de Lactobaccillus gasseri est de 2x10E9 - 9x10E9 ufc/g.

5. Capsule selon l'une quelconque des revendications précédentes **caractérisée en ce que** la quantité d'Enterococcus faecium est de 2x10E9-9x10E9 ufc/g.

6. Capsule selon l'une quelconque des revendications précédentes **caractérisée en ce que** le polysaccharide est le pullulane ou un dérivé de pullulane.

7. Composition consistant en Saccharomyces boulardii en une quantité de 10E8 - 10E9 unités/g et en au moins une bactérie choisie dans le groupe consistant en Lactobacillus crispatus, Lactobaccillus jensinii et Lactobaccillus gasseri et Enterococcus faecium, en une quantité de 10E8-10E9 ufc/g, pour son utilisation comme moyen pour traiter les vaginites.
